(19) Europäisches Patentamt
European Patent Office
Office européen des brevets

(11) Numéro de publication: 0 359 627 A1

(12) DEMANDE DE BREVET EUROPEEN

(21) Numéro de dépôt: 89402425.6

(22) Date de dépôt: 06.09.89

(51) Int. Cl.5: C 07 D 265/22
C 07 D 265/12,
C 07 D 498/04, A 61 K 31/535
//(C07D498/04,317:00,265:00)

(30) Priorité: 08.09.88 FR 8811746

(43) Date de publication de la demande:
21.03.90 Bulletin 90/12

(84) Etats contractants désignés:
AT BE CH DE ES FR GB GR IT LI LU NL SE

(71) Demandeur: PIERRE FABRE MEDICAMENT
125, Rue de la Faisanderie
F-75116 Paris (FR)

(72) Inventeur: Rieu, Jean-Pierre
Le Vixière Haute Avenue du Sidobre
F-081100 Castres (FR)

(74) Mandataire: Ahner, Francis et al
CABINET REGIMBEAU 26, avenue Kléber
F-75116 Paris (FR)

(54) **Nouveaux derives de dihydro-2,3 arylalcoylaminoalcoyl-3 4H-benzoxazine-1,3 ones-4 leur préparation et leur application en tant que médicaments utiles en thérapeutique.**

(57) L'invention concerne les nouveaux dérivés des arylacoylaminoalcoyl-3 4H-benzoxazine-1,3 one-4 de formule générale I

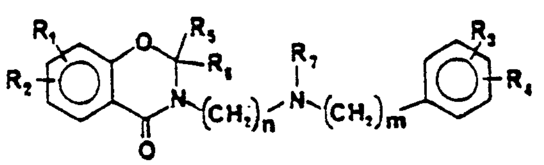

I

dans laquelle :
$R_1$, $R_2$, $R_3$, $R_4$ égaux ou différents représentent un hydrogène, un groupement alcoyle léger, ramifié ou non, alcoyloxy, halogéno, nitro, amino, acylamino, dialcoylamino et, $R_1$ et $R_2$ d'une part, $R_3$, $R_4$ d'autre part, peuvent fusionner pour donner un groupement divalent et plus particulièrement $-OCH_2O-$, $-OCH_2CH_2O-$ et $-CH=CH-CH=CH-$.
$R_5$ et $R_6$ égaux ou différents représentent un hydrogène, un groupement alcoyle ramifié ou non, saturé ou non, renfermant de 1 à 15 atomes de carbone, un groupement cycloalcoyle et $R_5$ et $R_6$ peuvent fusionner pour donner le groupement $-(CH_2)_p-$ avec p = 2 à 7.
$R_7$ représente un hydrogène, un groupement alcoyle ramifié ou non saturé ou non renfermant de 1 à 15 atomes de carbone, un groupement cycloalcoyle, un aryle substitué ou non et un arylalcoyle substitué ou non.
Les valeurs de m et n égales ou différentes peuvent varier de 1 à 4 inclus ainsi que les sels organiques ou minéraux de I thérapeutiquement acceptables.

EP 0 359 627 A1

## Description

### Nouveaux dérivés de dihydro-2,3arylalcoylaminoalcoyl-3 4H-benzoxazine -1,3 ones-4, leur préparation et leur application en tant que médicaments utiles en thérapeutique.

La présente invention réalisée au Centre de Recherche PIERRE FABRE a pour objet la synthèse de dérivés d'alcoylaminoalcoyl-3 benzoxazine-1,3 one-4 de structure originale I ou leurs sels et leur utilisation en tant que médicament notamment en thérapeutique cardiovasculaire.

Contrairement à leurs analogues azotés (quinazolones) les benzoxazine-1,3 one-4 ont été relativement peu étudiées. Seuls, quelques dérivés surtout substitués en 2 ont été revendiqués et ont abouti à la commercialisation de la chlorthénoxazine qui a été utilisée comme antiphlogistique (cf. brevet US 2 943 O87, CA 54, 2 4818 i (196O), Kadatz R., Arzneim. Forsch. 7 651 (1957).
Les activités de ces dérivés sont du même ordre que celles du salicylamide précurseur.
Quelques autres dérivés substitués sur le cycle aromatique et sur l'azote n'ont pas donné de résultat pharmacolgique marquant (cf. J. Finkelstein et E. Chiang J. Med. Chem. 11. 1O38 (1968) et notamment la (diéthylamino-2 éthyl)-3 dihydro-2,3 4H-benzoxazine-1,3 one-4 qui était inactive). La substitution sur l'azote de la dihydro-2,3 4H-benzoxazine-1,3 one-4 par un groupement arylalcoylaminoalcoyle conduit à des dérivés de structure originale I qui présentent une puissante activité bradycardisante et antiarythmique associée à un effet anti-ischémique important et inhibiteur calcique moyen.

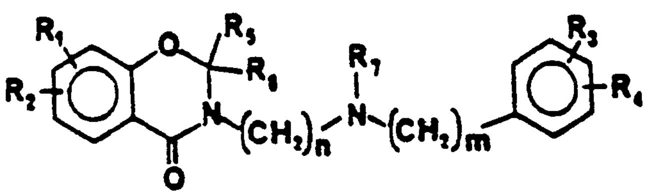

I

**Molécules revendiquées**

Les dérivés originaux de structure I objets de la présente invention sont définis comme suit :

. $R_1$, $R_2$, $R_3$, $R_4$ égaux ou différents représentent un hydrogène, un groupe alcoyle inférieur en $C_{1-6}$ ramifié ou non, un alcoyloxy, un halogène, un groupe nitro,amino, acylamino,dialcoylamino et,à titre d'exemple non limitatif : H, Me, Et, i-Pr, MeO, EtO, Br, Cl, F, $NO_2$, $NH_2$, $CH_3CONH$, $Me_2N$, enfin $R_1R_2$ d'une part et $R_3R_4$ d'autre part, peuvent fusionner deux à deux pour former un groupement $-OCH_2O-$, $OCH_2CH_2O$-ou -CH = CH-CH = CH-

. $R_5$ et $R_6$ égaux ou différents représentent un hydrogène, un groupement alcoyle ramifié ou non, saturé ou non, renfermant de 1 à 15 atomes de carbone, un groupement cycloalcoyle renfermant de 3 à 8 atomes de carbone et, en particulier, les radicaux H, Me, Et, i-Pr, n-heptyle, n-undécyle, n-pentadécyle, allyle, cyclohexyle. Enfin $R_5$ et $R_6$ peuvent fusionner pour donner un groupement polyméthylène $-(CH_2)_p-$ avec p pouvant prendre les valeurs de 2 à 7.

. $R_7$ représente un hydrogène, un groupement alcoyle ramifié ou non saturé ou non renfermant de 1 à 6 atomes de carbone, un groupement cycloalcoyle comportant de 3 à 8 atomes de carbone, un groupement aryle formé de 6 à 10 atomes de carbone substitué ou non, un groupement arylalcoyle renfermant de 7 à 15 atomes de carbone et, en particulier, les radicaux H, Me, Et, i-Pr, n-hexyle, allyle, cyclohexyle, phényle, benzyle, phénéthyle, méthoxy-4 phényle, diméthoxy-3,4 phénéthyle, chloro-4 phénéthyle.

. Les valeurs de m et n égales ou différentes peuvent varier de 1 à 4 inclus.

La présente invention inclut aussi les sels minéraux ou organiques thérapeutiquement acceptables du composé revendiqué I qui sont solubles dans l'eau et permettent d'administrer le médicament sous forme injectable lorsque celle-ci est présente.Les sels suivants sont donnés à titre d'exemple non limitatif : chlorhydrate, bromhydrate, mésylate, sulfate, phosphate, succinate, maléate, fumarate, citrate, tartrate, lactate, pamoate, pyroglutamate ou analogues.

La présente invention concerne également l'utilisation des composés de formule générale I à titre de médicament et les compositions pharmaceutiques renfermant ce médicament. Les compositions pharmaceutiques selon la présente invention peuvent utiliser un ou plusieurs composés de formule I éventuellement en association avec un ou plusieurs principes actifs. Enfin, les procédés de synthèse des composés de formule générale I font aussi partie de la présente invention.

Synthèse des composés de structure générale I

Deux voies générales de synthèse peuvent être utilisées pour préparer les composés de formule I :

**a) cyclisation finale**

**Méthode A**

La cyclisation finale est conduite à partir des salicylamides précurseurs de formule générale II par cyclocondensation avec le formaldéhyde sous forme de trioxyméthylène, paraformaldéhyde ; le paraldéhyde ou un aldéhyde aliphatique, un aldéhyde arylaliphatique, aromatique, cyclique, une cétone activée ou non III sous forme de cétal, selon l'équation A :

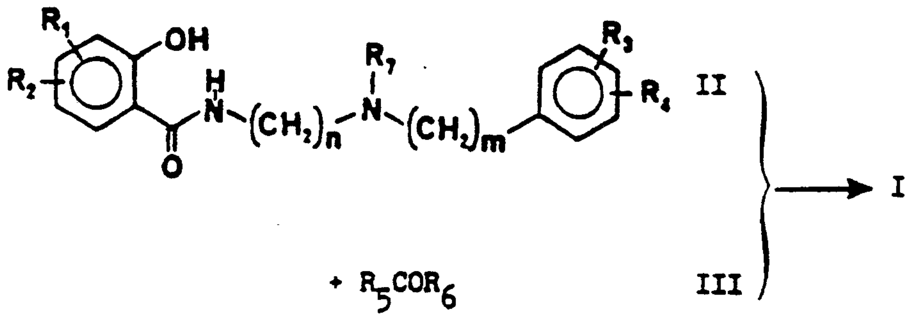

II

III

I

(A)

Dans les intermédiaires de formules générales II et III $R_1$ à $R_7$, m et n ont la même signification que dans le composé final I.

La réaction doit être réalisée en milieu acide fort comme par exemple : l'acide chlorhydrique pour activer le carbonyle et dans un solvant organique protique et de préférence dans l'acide acétique. Il est souhaitable d'utiliser un cosolvant aprotique comme le chloroforme ou mieux l'acétate d'éthyle dans le cas où l'acide salicylique est substitué par des groupments fragiles. La réaction est réalisée entre 25 et 50° et la durée de celle-ci varie de 15 mn à 2 h. Les salicylamides sont préparés soit par trans-amidification, soit par condensation directe des acides salicyliques avec les diamines convenablement substituées, en activant les acides sous forme de chlorure ou d'anhydrides mixte ou en utilisant un agent deshydratant comme la DCC en présence ou non d'HOBT.

**Méthode B**

Lorsque le radical $R_5$ ou $R_6$ est un aromatique directement lié au carbonyle, il est préférable de préparer la base de Schiff obtenue par réaction de ce dérivé carbonylé avec la diamine correspondante et de la condenser avec le chlorure d'acide salicylique convenablement substitué selon la réaction B :

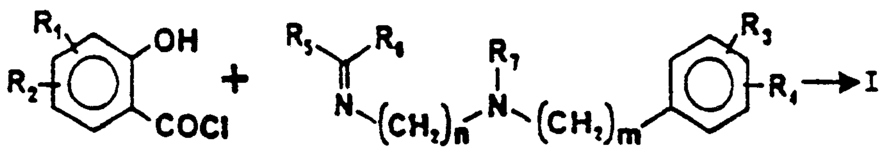

(B)

VI VII

Dans les intermédiaires de formule générale VI et VII, $R_1$ à $R_4$, $R_7$, m et n ont la même signification que dans le composé de formule I.

La condensation est réalisée de préférence à chaud dans un solvant apolaire comme le toluène.

**b) alkylation finale**

**Méthode C**

L'introduction de la fonction amine peut se faire à la fin par condensation de l'alcool activé IV avec l'amine V selon la réaction C :

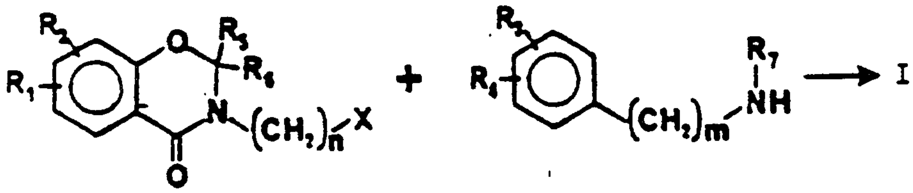


(C)

IV V

où $R_1$ à $R_7$, n et m ont la même signification que dans I et où X représente un halogène (Cl, Br, I) ou le groupement mésylate ($MeSO_3$-) ou tosylate ($CH_3C_6H_4SO_3$-). La condensation est réalisée de préférence à 25° dans le DMF en présence d'amine tertiaire. Cette méthode permet de préparer en particulier les composés I dans lesquels $R_7 = H$. On peut obtenir le composé final tri substitué sur l'azote soit directement, soit à partir de l'amine secondaire I ($R_7 = H$) par alcoylation avec un groupement $R_7$-X, où X représente un halogène ou un groupement sulfate, soit en alcoylant selon la réaction d'Eschweiller-Clarke par le couple acide formique-aldéhyde dérivé de $R_7$.

Les intermédiaires IV de structure originale font aussi l'objet de la présente invention.

Les bases précédentes peuvent être salifiées avec une quantité équimoléculaire d'acide souhaité en utilisant un solvant apolaire ou polaire comme l'acétate d'éthyle ou l'éthanol à titre d'exemple non limitatif.

**Exemple 1 (F 3151) : Chlorhydrate de dihydro-2,3 ((méthyl diméthoxy-3,4 phénéthyl-amino)-3 propyl)-3 4H-benzoxazine-1,3 one-4 (composé A)**

Une solution de 2 g (5,37 mmoles) de N-((méthyl, diméthoxy-3,4 phénéthylamino)-3 propyl) salicylamide dans 18 ml de chloroforme et 1 ml d'acide acétique est traitée pendant 2 mn avec un courant d'acide chlorhydrique gazeux. Trois cent quarante milligrammes de trioxyméthylène sont ensuit ajoutés au mélange précédent et on chauffe 2O mn à 7O-75° C. Après retour à température ordinaire, on fait passer un courant d'azote pendant 3O mn puis le mélange est filtré sur fritté et évaporé à siccité. Le résidu est repris dans du toluène et réévaporé à siccité à l'évaporateur rotatif sous vide. Le résidu solide (3,1O g) est trituré dans l'éther isopropylique, filtré. La poudre crème obtenue (m = 2,1 g) est recristallisée dans un mélange i-PrOH-EtOH 75-25 pour donner 1,53 g (Rdt = 68 %) de composé A de formule :

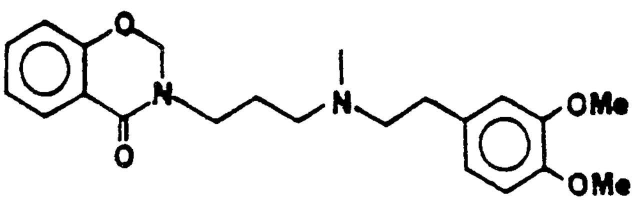

, HCl

**Composé A**

- Formule brute : $C_{22}H_{29}ClN_2O_4$
- Masse moléculaire : 42O,94
- Cristaux blancs
- Point de fusion : 167-169°C
- Chromatographie sur couche mince de gel de silice 6O Merck F 254
. éluant $CHCl_3$-MeOH-$NH_4OH$ : 90-09-01
. Rf = 0,57
- IR (KBr) $\nu$CO amide : 1650 $m^{-1}$
- RMN ($CDCl_3$) $\delta$ : 5,17 (s, 2H, - 0$C\underline{H}_2$N-), 2,75 (d. 3H, $C\underline{H}_3$-NH-)
- Solubilité : soluble à 1O % dans l'eau.

**Exemple 2 (F 3226) : Chlorhydrate de dihydro-2,3 diméthoxy-6,7 ((méthyl diméthoxy-3,4 phénéthylamino)-3 propyl)-3 4H-benzoxazine-1,3 one-4 (composé B)**

Un mélange de 1,5 g (3,5 mmoles) de diméthoxy-3,4 N-((méthyl diméthoxy-3,4 phénéthylamino)-3 propyl) salicylamide dans 2O ml d'acide acétique et 25 ml d'une solution 2N de HCl dans AcOEt est traitée avec 1,1 g de trioxyméthylène. Après 3 h d'agitation à 2O° C, le mélange est porté sur bain d'huile à 5O° pendant 2 h puis évaporé à siccité à l'évaporateur rotatif sous vide. Le résidu solide (m = 1,9 g) est purifié directement sur colonne de silice (6O g) en éluant avec un mélange $CHCl_3$-MeOH-$NH_4OH$ : 95-4,5-0,5. Les fractions renfermant le dérivé attendu sont réunies, évaporées à siccité (m = 1,7 g) puis le résidu est repris dans un mélange alcool isopropylique-acétate d'éthyle et chlorhydraté avec une solution d'HCl 2N dans AcOEt pour donner 1,2 g (Rdt = 40 %) du composé B de formule :

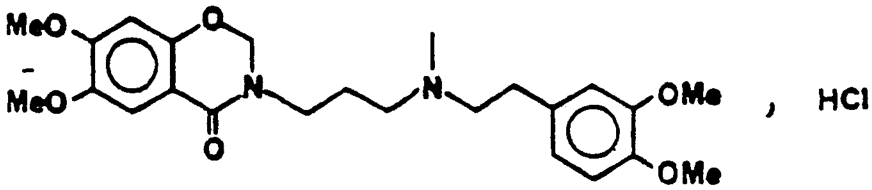

**Composé B**

- Formule brute : $C_{24}H_{33}ClN_2O_6$
- Masse moléculaire : 480.99
- Cristaux blancs
- Point de fusion : 174°C
- Chromatographie sur couche mince de gel de silice 60 Merck F 254
. éluant : $CHCl_3$-MeOH-$NH_4OH$ : 90.09.01
. Rf : 0,55
- IR (KBr) : $\nu$CO amide : 1650 $cm^{-1}$
- RMN ($CDCl_3$) $\delta$ : 2,6 (s, 3H, $C\underline{H}_3NH$), 5,05 (s, 2H, -$OC\underline{H}_2N$-)
- Solubilité : soluble à plus de 10 % dans l'eau.

**Exemple 3 (F 3262.01) : Chlorhydrate de dihydro-2,3 ((méthyl diméthoxy -3,4 phénéthylamino)-3 propyl)-3 méthoxy-8 4H benzoxazine-1,3 one-4 (composé C)**

En partant du méthoxy-3 N-((méthyl diméthoxy-3,4 phénéthylamino)-3 propyl) salicylamide et en utilisant le mode opératoire de préparation décrit pour le dérivé B, on obtient avec un rendement de 25 % le composé C de formule :

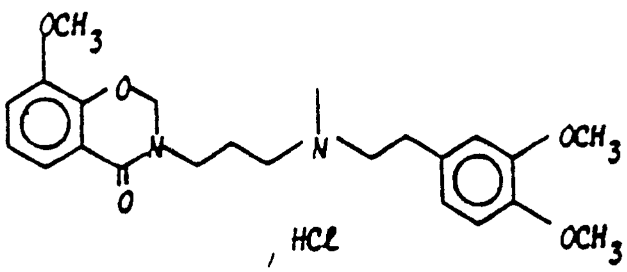

**Composé C**

- Formule brute : $C_{23}H_{31}ClN_2O_5$
- Masse moléculaire : 450.96
- Cristaux blancs
- Point de fusion : 143°C
- Chromatographie sur couche mince de gel de silice 60 Merck F 254
. éluant : $CHCl_3$-MeOH-$NH_4OH$ : 90.09.01
. Rf : 0,65
- IR (KBr) : $\nu$CO amide : 1650 $cm^{-1}$
- RMN ($CDCl_3$) $\delta$ : 2,75 (s, 3H, $C\underline{H}_3NH$-), 5,15 (s, 2H, -$OC\underline{H}_2H$)
- Solubilité : soluble à 10 % environ dans l'eau.

**Exemple 4 (F 3297) : Chlorhydrate de dihydro-2,3 ((méthyl diméthoxy -3,4 phénéthylamino)-3 propyl)-3 méthoxy-6 4H-benzoxazine-1,3 one-4 (composé D)**

En partant du méthoxy-5 N-((méthyl diméthoxy-3,4 phénéthylamino-3 propyl) salicylamide et en utilisant le mode opératoire de synthèse décrit pour B, on obtient avec un rendement de 56 % le composé D de structure :

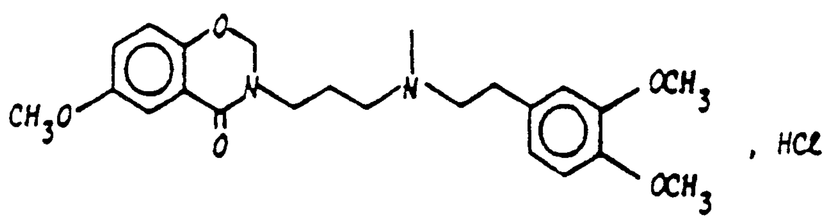

**Composé D**

- Formule brute : $C_{23}H_{31}ClN_2O_5$
- Masse moléculaire : 450,96
- Cristaux blancs
- Point de fusion : 142°C
- Chromatographie sur couche mince de gel de silice 60 Merck F 254
. éluant : $CHCl_3$-MeOH-$NH_4OH$ : 90-09-1
. Rf : 0,70
- IR (KBr) : $\nu$CO amide : 1660 $cm^{-1}$
- RMN ($CDCl_3$) $\delta$ : 2,85 (d, 3H, $\underline{CH_3}NH$), 5,05 (s, 2H, -O$\underline{CH_2}$N-)
- Solubilité dans l'eau : > 10 %

**Exemple 5 (F 3311) : Chlorhydrate de dihydro-2,3 ((méthyl diméthoxy -3,4 phénéthylamino)-3 propyl)-3 méthoxy-7 4H-benzoxazine-1,3 one-4 (composé E)**

Un mélange formé de 1,35 g (4,2 mmoles) de dihydro-2,3 méthoxy-7 ((méthanesulfonyloxy)-3 propyl)-3 4H-benzoxazine-1,3 one-4 de formule :

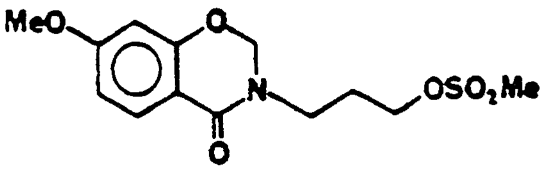

Formule brute : $C_{13}H_{17}NO_6$
Masse moléculaire : 315,34
Cristaux blancs
RMN ($CDCl_3$) $\delta$ : 2,95 (s, 3H, $\underline{CH_3}SO_3$), 3,7 (s, 3H, $\underline{CH_3}O$). 5,0 (s, 2H, -O$\underline{CH_2}$N-)
et de 1,46 g (6,3 mmoles) de chlorhydrate de N-méthylhomovératrylamine dans 10 ml de triéthylamine est dilué avec 20 ml de DMF RP et est porté sur bain d'huile à 50° pendant 10 h. Le mélange brut est dilué avec de l'eau et extrait plusieurs fois avec l'acétate d'éthyle puis la phase organique ainsi obtenue est lavée à l'eau, à l'eau salée, séchée sur sulfate et évaporée à siccité. Le résidu (1,2 g) est purifié sur une colonne ouverte de silice (25 g) en éluant avec un mélange $CHCl_3$-MeOH-$NH_4OH$ : 95-04,5-00,5. Les fractions renfermant le composé attendu sont réunies et évaporées à siccité puis l'huile résiduelle est reprise dans AcOEt et chlorhydratée avec une solution 2N HCl dans AcOEt pour donner 550 mg (Rdt : 30 %) de composé E de formule :

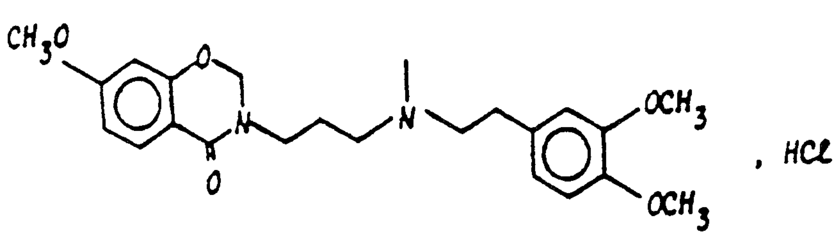

**Composé E**

- Formule brute : $C_{23}H_{31}ClN_2O_5$
- Masse moléculaire : 450,96
- Cristaux blancs
- Point de fusion : 148°C
- Chromatographie sur couche mince de gel de silice 60 Merck F 254
. éluant : $CHCl_3$-MeOH-$NH_4OH$ : 90-09-01
. Rf : 0,61

- IR (KBr) : $\nu$CO amide : 1665 $cm^{-1}$
- RMN ($CDCl_3$) δ : 2,75 (d, 3H, C$\underline{H}_3$NH), 5,05 (s, 2H, -0C$\underline{H}_2$N-)
- Soluble dans l'eau à 10 %

**Exemple 6 (F 3265) : Chlorhydrate de dihydro-2,3 ((méthyl diméthoxy -3,4 phénéthylamino)-3 propyl)-3 méthyl-6 4H-benzoxazine-1,3 one-4 (composé F)**

En partant du méthyl-5 N-((méthyl diméthoxy-3,4 phénéthylamino)-3 propyl) salicylamide et en utilisant le procédé de synthèse décrit dans l'exemple 2, on obtient avec un rendement de 57 % le composé F de structure :

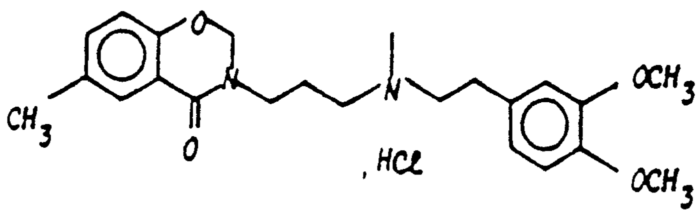

**Composé F**

- Formule brute : $C_{23}H_{31}ClN_2O_4$
- Masse moléculaire : 434,96
- Cristaux blancs
- Point de fusion : 147° C
- Chromatographie sur couche mince de gel de silice 60 Merck F 254
. éluant : $CHCl_3$-MeOH-$NH_4OH$ : 90-09-01
. Rf : 0,65
- IR (KBr) : $\nu$CO amide : 1650 $cm^{-1}$
- RMN ($CDCl_3$) δ : 2,8 (s, 3H, C$\underline{H}_3$NH), 5,06 (s, 2H, 0C$\underline{H}_2$H)
- Solubilité dans l'eau voisine de 10 %.

**Example 7 (F 3266) : Chlorhydrate de dihydro-2,3 ((méthyl diméthoxy-3,4 phénéthylamino)-3 propyl)-3 méthyl-7 4H-benzoxazine-1,3 one-4 (composé G)**

A partir du méthyl-4 N-((méthyl diméthoxy-3,4 phénéthylamino-3) propyl) salicylamide et en utilisant le procédé de synthèse décrit pour dans l'exemple 2, on obtient avec un rendement de 35 % le composé G de structure :

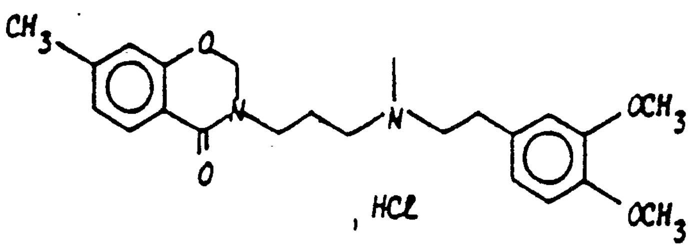

**Composé G**

- Formule brute : $C_{23}H_{31}ClN_2O_4$
- Masse moléculaire : 434,96
- Cristaux blancs
- Point de fusion : 148°C
- Chromatographie sur couche mince de gel de silice 60 Merck F 254
. éluant : $CHCl_3$-MeOH-$NH_4OH$ : 90-09-01
. Rf : 0,59
- IR (KBr) : $\nu$CO amide : 1660 $cm^{-1}$
- RMN ($CDCl_3$) δ : 2,8 (s, 3H, C$\underline{H}_3$NH), 5,08 (s, 2H, -0C$\underline{H}_2$N-)
- Soluble dans l'eau à 9 %

**Exemple 8 (F 3267) : Chlorhydrate de dihydro-2,3 ((méthyl diméthoxy-3,4 phénéthylamino)-3 propyl)-3 méthyl-8 4H-benzoxazine-1,3 one-4 (composé H)**

D'une manière analogue au procédé décrit dans l'exemple 2 et en utilisant le méthyl-3 N-((méthyl diméthoxy-3,4 phénéthylamino)-3 propyl) salicylamide, on prépare avec un rendement de 50 % le composé H

de structure :

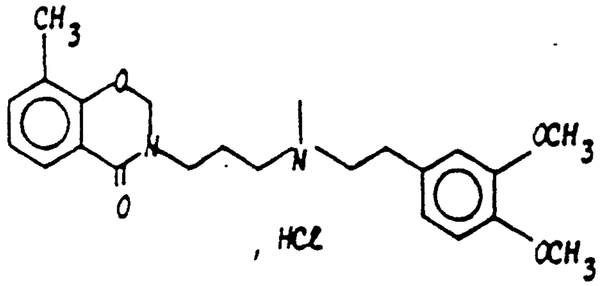

**Composé H**

- Formule brute : $C_{23}H_{31}ClN_{2}O_{4}$
- Masse moléculaire : 434,96
- Cristaux blancs
- Point de fusion : 149°C
- Chromatographie sur couche mince de gel de silice 60 Merck F 254
  . éluant : $CHCl_3$-MeOH-$NH_4OH$ : 90-09-01
  . Rf : 0,60
- IR (KBr) : ν CO amide : 1660 $cm^{-1}$
- RMN ($CDCl_3$) δ : 2,8 (s, 3H, $C\underline{H}_3NH$), 5,1 (s, 2H, -$OC\underline{H}_2N$-)
- Soluble à 8 % dans l'eau

**Exemple 9 (F 3263) : Chlorhydrate de chloro-7 dihydro-2,3 ((méthyl diméthoxy-3,4 phénéthylamino)-3 propyl)-3 4H-benzoxazine-1,3 one-4 (composé J)**

En utilisant le chloro-4 N-((méthyl diméthoxy-3,4 phénéthylamino)-3 propyl) salicylamide et en employant le procédé décrit dans l'exemple 2, on obtient avec un rendement de 35 % le composé J de structure :

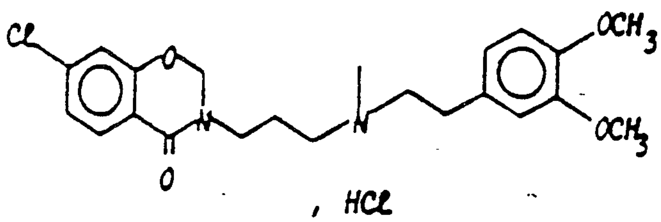

**Composé J**

- Formule brute : $C_{22}H_{28}Cl_{2}N_{2}O_{4}$
- Masse moléculaire : 455,38
- Cristaux blancs
- Point de fusion : 158°C
- Chromatographie sur couche mince de gel de silice 60 Merck F 254
  . éluant : $CHCl_3$-MeOH-$NH_4OH$ : 90-09-01
  . Rf : 0,64
- IR (KBr) : ν CO amide : 1660 $cm^{-1}$
- RMN ($CDCl_3$) δ : 2,83 (s, 3H, $C\underline{H}_3NH$), 5,2 (s, 2H, -$OC\underline{H}_2$-N-)
- Soluble à 12 % dans l'eau

**Exemple 10 (F 3264) : Chlorhydrate de bromo-6 dihydro-2,3 ((méthyl (diméthoxy-3,4 phénéthylamino)-3 propyl)-3 4H-benzoxazine-1,3 one-4 (composé K)**

En partant du bromo-5 N-((méthyl diméthoxy-3,4 phénéthylamino)-3 propyl) salicylamide et en utilisant le procédé de préparation de l'exemple 2, on prépare avec un rendement de 45 % le composé K de structure générale :

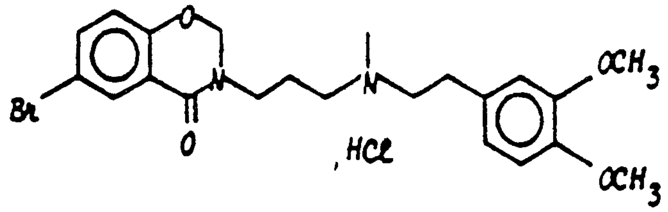

**Composé K**

- Formule brute : $C_{22}H_{28}BrClN_2O_4$
- Masse moléculaire : 499,84
- Cristaux blancs
- Point de fusion : 159°C
- Chromatographie sur couche mince de gel de silice 60 Merck F 254
. éluant : $CHCl_3$-MeOH-$NH_4OH$ : 90-09-01
. Rf : 0,68
- IR (KBr) : $\nu$CO amide : 1660 $cm^{-1}$
- RMN ($CD_3OD$) $\delta$ : 2,78 (s, 3H, C<u>H</u>3NH), 5,07 (s, 2H, -OC<u>H</u>2N-)
- Soluble à 9 % dans l'eau

**Exemple 11 (F 3296) : Chlorhydrate de dihydro-2,3 ((méthyl diméthoxy-3,4 phénéthylamino)-3 propyl)-3, méthoxy-8 méthyl-2 4H-benzoxazine-1,3 one-4 (composé L)**

En partant du méthoxy-3 N-((méthyl diméthoxy-3,4 phénéthylamino)-3 propyl) salicylamide en remplaçant le trioxyméthylène par le paraldéhyde et en utiisant le procédé de préparation de l'example 2, on prépare avec un rendement de 55 % le composé L de formule :

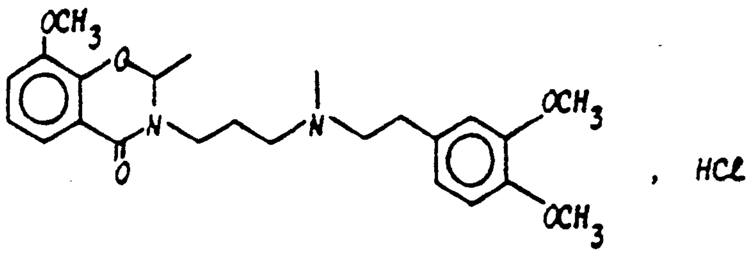

**Composé L**

- Formule brute : $C_{24}H_{33}ClN_2O_5$
- Masse moléculaire : 464,99
- Cristaux blancs
- Point de fusion : 158°C
- Chromatographie sur couche mince de gel de silice 60 Merck F 254
. éluant : $CHCl_3$-MeOH-$NH_4OH$ : 90-09-01
. Rf : 0,72
- IR (KBr) : $\nu$CO amide : 1665 $cm^{-1}$
- RMN ($CDCl_3$) $\delta$ : 1,9 (d, 3H, C<u>H</u>3CH), 2,8 (d, 3H, C<u>H</u>3NH), 5,4 5m, 1H, -<u>CH</u>-CH3)
- Soluble à 10 % dans l'eau

**Exemple 12 (F 3298) : Chlorhydrate de dihydro-2,3 diméthoxy-6,7 ((méthyl diméthoxy-3,4 phénéthylamino)-3 propyl)-3 méthyl-2 4H-benzoxazine-1,3 one-4 (composé M)**

En appliquant le mode opératoire de l'exemple 2 et en utilisant le même salicylamide substitué, mais en remplaçant le trioxyméthylène par le paraldéhyde, on prépare avec un rendement de 70 % le dérivé méthylé en 2 de B, c'est-à-dire le composé M de formule :

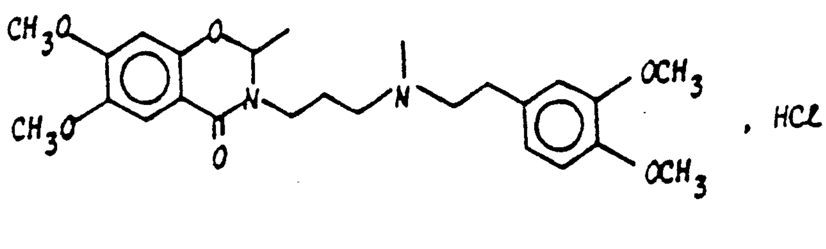

**Composé M**

- Formule brute : $C_{25}H_{35}ClN_2O_6$
- Masse moléculaire : 495,016
- Cristaux blancs
- Point de fusion : 149°C
- Chromatographie sur couche mince de gel de silice 60 Merck F 254
. éluant : $CHCl_3$-MeOH-$NH_4OH$ : 90-09-01
. Rf : 0,70
- IR (KBr) : $\nu$CO amide : 1655 $cm^{-1}$
- RMN ($CDCl_3$) $\delta$ : 1,85 (d, 3H, C<u>H</u>$_3$CH), 2,74 (d, 3H, C<u>H</u>$_3$NH), 5,25 (m, 1H, -C<u>H</u>CH$_3$)
- Soluble à 10 % dans l'eau

**Exemple 13 (F 3299) : Chlorhydrate de dihydro-2,3 diméthoxy-6,7 ((méthyl diméthoxyphénéthylamino)-3 propyl)-3 n-heptyl-2 4H-benzoxazine-1,3 one-4 (composé N)**

En utilisant le mode opératoire de l'exemple 2 et en partant du même salicylamide substitué, mais en remplaçant le trioxyméthylène par le n-octanal, on prépare avec un rendement de 58 % le dérivé N de formule :

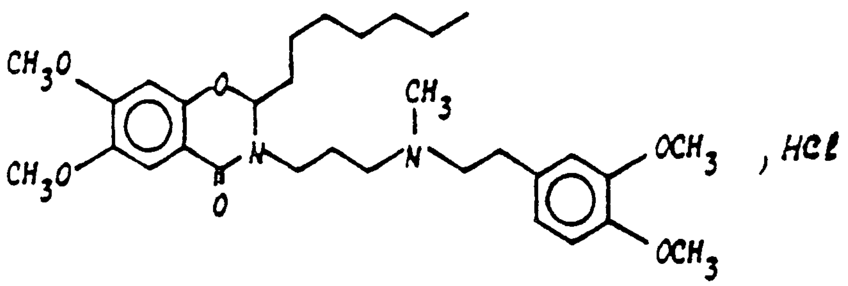

**Composé N**

- Formule brute : $C_{31}H_{47}ClN_2O_6$
- Masse moléculaire : 579,186
- Cristaux beige clair
- Point de fusion : 117°C
- Chromatographie sur couche mince de gel de silice 60 Merck F 254
. éluant : $CHCl_3$-MeOH-$NH_4OH$ : 90-09-01
. Rf : 0,75
- IR (KBr) : $\nu$CO amide : 1660 $cm^{-1}$
- RMN ($CDCl_3$) $\delta$ : 2,75 (d, 3H, C<u>H</u>$_3$NH), 5,2 (m, 1H, -CHC<u>H</u>$_2$-)
- Soluble à 10 % dans l'eau

**Exemple 14 (F 3310) : Chlorhydrate de dihydro-2,3 diméthoxy-6,7 ((méthyl phénéthylamino)-3 propyl)-3 4H-benzoxazine-1,3 one-4 (composé 0)**

A partir du diméthoxy-4,5 N-(méthylphénéthylamino-3 propyl) salicylamide et en utilisant le mode opératoire décrit dans l'exemple 9 sans purifier sur colonne, on obtient directement avec un rendement de 85 % le composé 0 de structure :

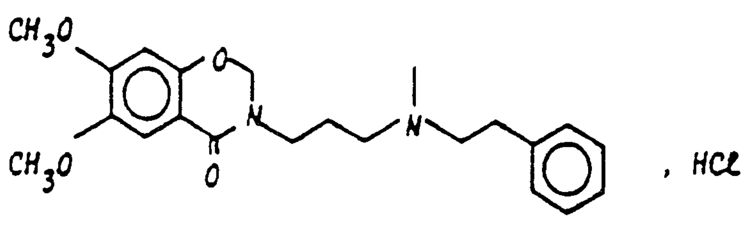

**Composé 0**

- Formule brute : $C_{22}H_{29}ClN_2O_4$
- Masse moléculaire : 420,946
- Cristaux blancs
- Point de fusion : 174°C
- Chromatographie sur couche mince de gel de silice 60 Merck F 254

. éluant : $CHCl_3$-MeOH-$NH_4OH$ : 90-09-01
. Rf : 0,65
- IR (KBr) : $\nu$CO amide : 1670 $cm^{-1}$
- RMN ($CDCl_3$) $\delta$ : 2,8 (s, 3H, $CH_3NH$), 5,06 (s, 2H, $-OCH_2N-$)
- Soluble à plus de 10 % dans l'eau

**Exemple 15 (F 3325) : Chlorhydrate de dihydro-2,3 diméthoxy-6,7 méthyl ((diméthoxy-3,4 phénéthylamino)-3 propyl)-3 4H-benzoxazine-1,3 one-4 (composé P)**

La dihydro-2,3 diméthoxy-6,7 [(méthanesulfonyloxy)-3 propyl]-3 4H-benzoxazine-1,3 one-4 de formule :

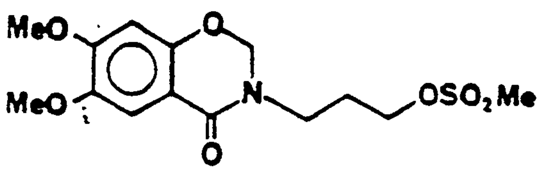

Formule brute : $C_{14}H_{19}NO_7S$
Masse moléculaire : 345,37
Cristaux blancs
Point de fusion : 123°C
IR (KBr) : $\nu$CO amide 1660 $cm^{-1}$, $\nu SO_3$ 1360, 1180 $cm^{-1}$
RMN ($CDCl_3$) $\delta$ : 2,73 (s, 3H, $CH_3SO_3$), 5 (s, 2H, $OCH_2N$)
est condensée avec un rendement de 70 % avec l'homovératrylamine en excès selon le mode opératoire décrit dans l'exemple 5 pour donner le composé P de formule :

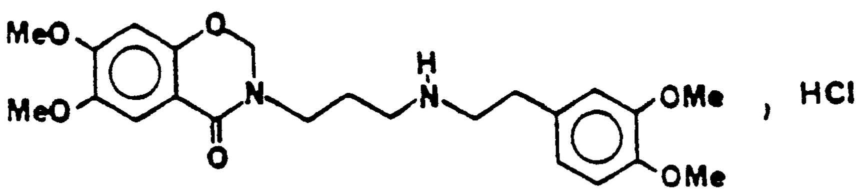

**Composé P**
- Formule brute : $C_{23}H_{31}ClN_2O_6$
- Masse moléculaire : 466,962
- Cristaux blancs
- Point de fusion : 155°C
- Chromatographie sur couche mince de gel de silice 60 Merck F 254
. éluant : $CHCl_3$-MeOH-$NH_4OH$ : 90-09-01
. Rf : 0,43
- IR (KBr) : $\nu$CO amide : 1645 $cm^{-1}$
- RMN ($CDCl_3$) $\delta$ : 5,00 (s, 2H, $-OCH_2N-$)
- Soluble à plus de 10 % dans l'eau

**Exemple 16 : Chlorhydrate de dihydro-2,3 diméthoxy-6,7 ((méthyl diméthoxy-3,4 phénéthylamino)-3 propyl)-3 4H-benzoxazine-1,3 one-4 (composé B)**

A partir de la dihydro-2,3 diméthoxy-6,7[(méthanesulfonyloxy)-3 propyl]-3 4H benzoxazine-1,3 one-4 intermédiaire préparée comme dans l'exemple 15 mais en la condensant sur la N-méthyl, diméthoxy-3,4 phénéthylamine comme dans l'exemple 15, on obtient avec un rendement de 68 % le composé B dont les caractéristiques physicochimiques sont identiques à celles décrites dans l'exemple 2.

**Exemple 17 : Monohydrate du chlorhydrate de dihydro-2,3 diméthoxy-6,7** ((benzyl diméthoxy-3,4 phénéthylamino)-3 propyl)-3 4H-benzoxazine-1,3 **one-4 (composé Q)**

Une solution de 2,2 g (4,7 mmoles) de chlorhydrate de dihydro-2,3 diméthoxy-6,7 ((diméthoxy-3,4 phénéthylamino)-3 propyl)-3 4H benzoxazine 1,3 one-4 préparée comme décrit dans l'exemple 15 dans 25 ml de DMF RP est refroidie sur bain de glace puis traitée avec 3 ml de triéthylamine puis avec 0,88 g (5,2 mmoles) de bromure de benzyle et agitée 1 h à 0°C puis 5 h à 25°C. Le mélange est évaporé à siccité sous vide puis repris dans 50 ml d'eau, refroidi et amené à pH 9 par addition de lessive de soude à 30 %. La base est extraite plusieurs fois à l'acétate d'éthyle. La phase organique ainsi obtenue est lavée à l'eau, à l'eau salée et séchée sur sulfate de sodium puis évaporée à siccité. L'huile résiduelle (2,45 g) est purifiée sur colonne de silice ouverte (50 g) en éluant avec un mélange $CHCl_3$-MeOH 95-05. Les fractions renfermant le dérivé attendu sont réunies, évaporées à siccité puis reprises dans l'éther et chlorhydratées avec une solution 2N d'HCl dans

AcOEt pour donner 1,75 g (Rdt : 68 %) de composé Q de formule :

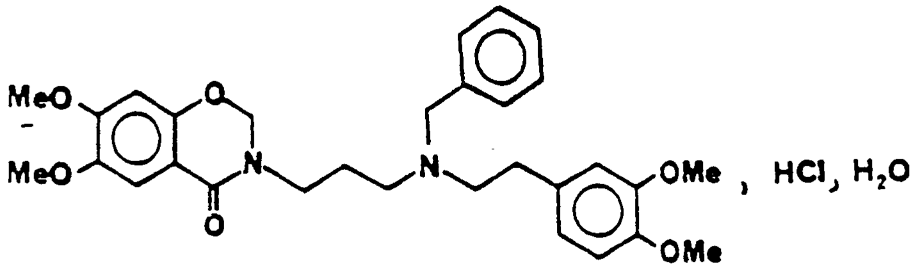

**Composé Q**

- Formule brute : $C_{30}H_{39}ClN_2O_7$
- Masse moléculaire : 575,10
- Poudre amorphe beige clair
- Point de fusion lent : 78-82°C
- Chromatographie sur couche mince de gel de silice 60 Merck F 254
  . éluant : $CHCl_3$-MeOH-$NH_4OH$ : 90-09-01
  . Rf : 0,76
- IR (KBr) : $\nu$CO amide : 1660 $cm^{-1}$
- Soluble à 6 % dans l'eau

**Exemple 18 : Chlorhydrate de dihydro-2,3 diméthoxy-6,7 ((méthyl** ((diméthoxy-3,4 phénéthylamino)-3 propyl)-3 4H-benzoxazine-1,3 one-4 **(composé B)**

8 g (18,5 mmoles) de base de composé P sont dissous en refroidissant dans 2,1 ml (56 mmoles) d'acide formique, agités 10 mn puis traités à 25°C avec 1,6 ml de formol à 40 % (23 mmoles) puis portés pendant 10 mn sur bain d'huile préchauffé à 80°C. Le mélange est refroidi, repris dans le chlorure de méthylène et basifié avec de la lessive de soude. Les phases sont séparées, la phase aqueuse est réextraite avec le même solvant, les phases organiques sont réunies, lavées à l'eau salée puis séchées et évaporées. Le résidu est chlorhydraté comme dans l'exemple 2 pour donner, avec un rendement de 90 %, le composé B possédant les mêmes caractéristiques que celles décrites dans l'exemple 2.

**Exemple 19 ( F 3326) : Chlorhydrate de dihydro-2,3 diméthoxy-6,7** ((diméthoxy-3,4 phénéthylamino)-2 éthyl)-3 4H-benzoxazine-1,3 one-4 **(composé R)**

A partir de la dihydro-2,3 diméthoxy-6,7 [(méthanesulfonyloxy)-2 éthyl]-3 4H-benzoxazinone de formule :

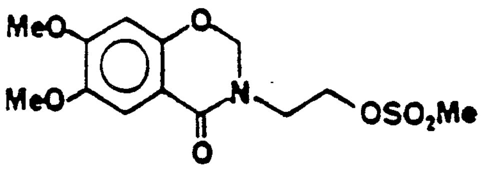

Formule brute : $C_{13}H_{17}NO_7S$
Masse moléculaire : 331,34
Cristaux blanc cassé
Point de fusion : 135°C
IR (KBr) : $\nu$CO 1660, $MeSO_3R$ 1350 et 1180 $cm^{-1}$
RMN ($CDCl_3$) $\delta$ : 2,8 (s, 3H, $CHSO_3$) ; 5,2 (s, 2H, -$OCH_2N$)

et en la condensant avec l'homovératrylamine en excès selon le procédé décrit dans l'exemple 15 ou 16, on obtient avec un rendement de 60 % le composé R de formule :

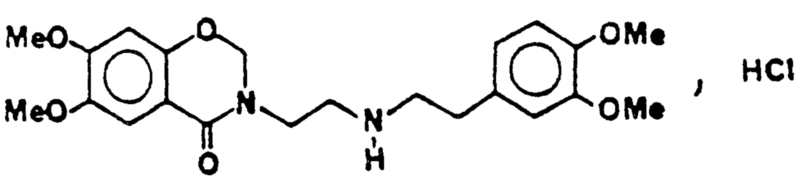

**Composé R**

- Formule brute : $C_{22}H_{29}ClN_2O_6$
- Masse moléculaire : 452.94

- Cristaux blancs
- Point de fusion : 198°C
- Chromatographie sur couche mince de gel de silice 60 Merck F 254
. éluant : $CHCl_3$-MeOH-$NH_4OH$ : 90-09-01
. Rf : 0,58
- IR (KBr) : $\nu$CO amide : 1665 $cm^{-1}$
- RMN ($CDCl_3$) $\delta$ : 5,2 (s, 2H, -O$C\underline{H}_2$N-)
- Soluble à 12 % dans l'eau

**Exemple 20 (F 3327) : Chlorhydrate de dihydro-2,3 diméthoxy-6,7 ((méthyl diméthoxy-3,4 phénéthylamino)-2 éthyl)-3 4H-benzoxazine-1,3 one-4 (composé S)**

A partir de l'intermédiaire précédent : la dihydro-2,3 diméthoxy-6,7 (méthanesulfonyloxy-2 éthyl)-3 4H-benzoxazine-1,3 one-4 mais en condensant ce produit avec la N-méthyl homovératrylamine selon le mode opératoire décrit dans l'exemple 5 ou 15, on prépare avec un rendement de 65 % le composé S de structure :

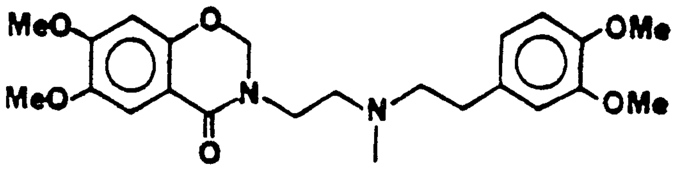 , HCl

**Composé S**

- Formule brute : $C_{23}H_{31}ClN_2O_6$
- Masse moléculaire : 466,96
- Cristaux blancs
- Point de fusion : 140°C
- Chromatographie sur couche mince de gel de silice 60 Merck F 254
. éluant : $CHCl_3$-MeOH-$NH_4OH$ : 90-09-01
. Rf : 0,65
- IR (KBr) : $\nu$CO 1665 $cm^{-1}$
- RMN ($CDCl_3$) $\delta$ : 2,83 (s, 3H, $C\underline{H}_3$NH) ; 5,2 (s, 2H, -O$C\underline{H}_2$N-)

**Expérimentations biologiques**

Les composés de la présente invention de formule générale I et leurs sels d'acides, thérapeutiquement acceptables, présentent d'intéressantes propriétés pharmacodynamiques. En effet, ces composés sont fortement bradycardisants et modérément anticalciques. Ils agissent directemment au niveau cardiaque et sont utilisés comme antiangoreux. Ils diminuent la fréquence cardiaque et la consommation d'oxygène.

**1) Toxicologie**

Les composés chimiques précédemment décrits ont été soumis à des contrôles de toxicité. Cette étude a été réalisée sur la souris conventionnelle pesant 20 à 22 g. Les sels des composés de formule générale I solubles dans l'eau ont été administrés par voie intra veineuse. La dose léthale 50 % ($DL_{50}$) est calculée selon la méthode de Miller et Tainter (Proc. Soc. Exp. Biol. Med. 1944. 57. 261). Les $DL_{50}$ relatives du composé A (exemple 1) et B (exemple 2) sont données ci-dessous à titre d'exemple non limitatif.

Tableau 1

| Composés | $DL_{50}$ iv |
|---|---|
| A | 27 mg/kg |
| B | 48 mg/kg |

**2) Etude pharmacologique**

Les expérimentations pharmacologiques auxquelles ont été soumises les molécules chimiques objet de la présente invention, ont permis de mettre en évidence une intéressante activité sur le système cardiovasculaire. Les activités pharmacologiques ont été mises en évidence in vivo et in vitro.

a) La technique de la ligature coronaire chez le rat anesthésié selon Clark et al. (J. Pharm. Methods 1980, 3, 357-368) a été utilisée pour mettre en évidence l'activité antidysrythmique.

Les résultats sont donnés dans le tableau II et exprimés par le pourcentage de variation par rapport aux témoins de la fréquence cardiaque 1 mn et 10 mn après l'injection des composés A, B, G, J et N relatifs aux exemples 1, 2, 7, 9 et 13 donnés à titre d'exemples non limitatifs, SC représente le pourcentage de réduction

des troubles du rythme induit par ces composés, n représente le nombre d'animaux.

Tableau II

| Composés (Nb rats) | Doses mg/kg iv | % baisse de fréquence cardiaque | | % de baisse des dysrythmies |
|---|---|---|---|---|
| | | après 1 mn | après 10 mn | |
| A (n=5) | 2,5 | - 30 | - 11 | - 9 |
| A (n=5) | 10 | - 50 | - 19 | - 76 |
| B (n=7) | 2,5 | - 50 | - 28 | - 38 |
| B (n=7) | 10 | - 66 | - 60 | - 80 |
| G (n=7) | 2,5 | - 39 | - 16 | - 1 |
| J (n=7) | 2,5 | - 32 | - 12 | - 37 |
| N (n=5) | 2,5 | - 4 | - 6 | - 72 |

b) L'effet bradycardisant et l'analyse des temps de conduction intracardiaque one été étudiés sur des rats anesthésiés au pentobarbital sodique (40 mg/kg ip) et laissés en ventilation spontanée.

L'électrocardiogramme est simultanément enregistré dans les dérivations standard 0I, 0II, 0III, la pression artérielle est mesurée au niveau d'une artère fémorale. Les pourcentages de variation sont considérés à l'acmé d'action. Les résultats relatifs aux composés A, B, J, G et N sont donnés dans le tableau III à titre d'exemple non limitatif.

Tableau III

| Composés (Nb rats) | Doses mg/kg iv | % PA | FC | % de modification des espaces de ECG | | | |
|---|---|---|---|---|---|---|---|
| | | | | PQ | QRS | QTC | TP |
| A (n=10) | 3 | -33 | -31 | +26 | +11 | +29 | +76 |
| B (n=7) | 1 | -16 | -32 | +9 | +12 | +38 | +95 |
| B (n=7) | 3 | -34 | -55 | +17 | +24 | +75 | +211 |
| G (n=5) | 1 | -10 | -18 | +10 | +5 | +12 | +32 |
| J (n=5) | 1 | -13 | -16 | +9 | +12 | +10 | +29 |
| N (n=5) | 1 | -9 | -12 | +7 | +12 | +10 | +21 |

PA : pression artérielle moyenne

FC : fréquence cardiaque

PQ : temps de conduction auriculo-ventriculaire

QRS : temps de conduction intraventriculaire

QTC : indice de Bazett

TP : diastole cardiaque électrique

L'effet bradycardisant est également retrouvé chez le chien vigile dont la fréquence cardiaque est élevée par l'injection d'hydralazine à la dose de 1 mg/kg iv selon la méthode de Kobinger et Lillie (Europ. J. Pharmacol. 1981, 72, 153-164). L'administration d'hydralazine engendre une tachycardie. Lorsque celle-ci est stabilisée, les produits à étudier sont injectés par voie intraveineuse.

Les résultats sont donnés dans le tableau IV pour les composés A et B à titre d'exemple non limitatif :

Tableau IV

| Composés n=nbre de chiens | Doses mg/kg iv | fréquence cardiaque(bts/mn) | | variations |
|---|---|---|---|---|
| | | avant | après | |
| A (n=2) | 1 | 215 | 140 | - 35 % |
| B (n=2) | 1 | 175 | 105 | - 40 % |

Il est à noter que les composés de la présente invention n'induisent pas de bradycardie sur le chien à rythme

normal. c) Enfin les activités chronotropes et inotropes ont été mesurées sur des oreillettes isolées de cobaye placées dans des cuves à organes isolés. Les organes sont alors reliés à des capteurs de force. L'oreillette droite battant en rythme spontané permet d'apprécier les effets chronotropes négatifs des produits. Quant aux oreillettes gauches, elles sont stimulées électriquement à rythme fixe de manière à évaluer les actions sur l'inotropisme.
Les pourcentages des variations sont répertoriés dans le tableau V pour les composés A, B, G et J à titre d'exemples non limitatifs.

Tableau V

| Composés | Concentrations µg/ml | Oreillette droite effet chronotrope | Oreillette gauche effet inotrope |
|---|---|---|---|
| A | 0,3 | - 15 | - 16 |
| A | 1 | - 19 | - 15 |
| B | 0,3 | - 27 | - 9 |
| B | 1 | - 64 | - 6,5 |
| G | 1 | - 35 | - 4 |
| J | 1 | - 29 | - 11 |

Les composés de la présente invention se caractérisent par un index favorable, l'effet bradycardisant l'emporte nettement sur le faible effet dépresseur cardiaque.

**3) Applications thérapeutiques**

Compte tenu de leur activité pharmacologique, les dérivés de la présente invention peuvent être utilisés en thérapeutique humaine et animale dans le traitement des troubles cardiovasculaires. Ces composés induisent une forte bradycardie associée à un effet antiarythmique. Cette bradycardie réduit la consommation d'oxygène et accroît le temps de perfusion diastolique et, à ce titre, ces composés sont utiles comme antiangoreux. Enfin, ils peuvent être utilisés comme antiischémiques car ils diminuent les troubles du rythme consécutifs à une ischémie.

Les composés de la présente invention sont utilisés pour préparer des médicaments pouvant être administrés chez les animaux à sang chaud ou chez l'homme. L'administration peut être réalisée par voie orale, parentérale ou rectale, chaque dose est constituée : d'un adjuvant pharmaceutique inerte facilitant la préparation et l'absorption du médicament et du principe actif pouvant être aussi associé à un autre. Ces médicaments peuvent se présenter sous forme de comprimés, gélules, suspensions, émulsions, sirops, suppositoires, solutions ou analogues. L'administration du principe actif de la présente invention se fait à une dose moyenne comprise entre O,1 et 5 mg/kg de poids du corps.
Trois préparations sont données à titre d'exemple non limitatif, les ingrédients ainsi que d'autres pouvant être introduits en d'autres proportions sans modifier la portée de l'invention.

**Exemple 21 : solution injectable**

Chlorhydrate de dihydro-2,3 diméthoxy-6,7 ((méthyl diméthoxy-3,4 phénéthylamino)-3 propyl)-3 4H-benzoxazine-1,3 one-4 : 5 mg
Solution de NaCl à 0,9 % dans l'eau distillée stérile : qsp 2 ml
stockée dans une ampoule en verre inactinique et à conserver à l'abri de la chaleur.

Exemple 22 : comprimés

| | |
|---|---|
| Chlorhydrate de dihydro-2,3 diméthoxy-6,7 ((méthyl diméthoxy-3,4 phénéthylamino)-3 propyl)-3 4H-benzoxazine-1,3 one-4 : | 50 mg |
| . lactose : | 15 mg |
| . amidon de maïs : | 4 mg |
| . polyvinyl pyrrolidone : | 5 mg |
| . stéarate de magnésium : | 1 mg |
| . carboxyméthyl cellulose : | 15 mg |
| poids total : | 90 mg |

**Exemple 23 : suppositoires**

Chlorhydrate de dihydro-2,3 diméthoxy-6,7 ((méthyl diméthoxy-3,4 phénéthylamino)-3 propyl)-3 4H-benzoxazine-1,3 one-4 : 120 mg
Base pour suppositoire (beurre de cacao) : qsp 2,5 g
à conserver à l'abri de la lumière, de la chaleur et de l'humidité.

**Revendications**

1) L'invention concerne les nouveaux dérivés de dihydro-2,3 arylalcoylaminoalcoyl-3 4H-benzoxazine-1,3 one-4 de formule générale I :

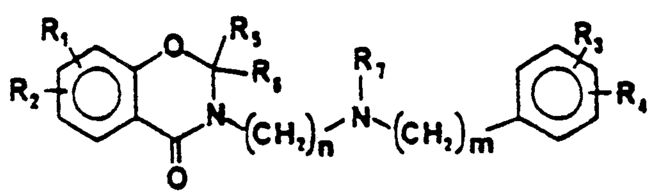

dans laquelle les substituants sont définis comme suit :

. $R_1$, $R_2$, $R_3$, $R_4$ égaux ou différents représentent un hydrogène, un groupement alcoyle inférieur en $C_{1-6}$, ramifié ou non, alcoyloxy, halogéno, nitro, amino, acylamino, dialcoylamino et, en particulier, les radicaux H, Me, Et, i-Pr, MeO, EtO, Br, Cl, F, $NO_2$, $NH_2$, $CH_3CONH$, $Me_2N$ ; enfin $R_1$ et $R_2$ d'une part, $R_3$ et $R_4$ d'autre part, peuvent fusionner deux à deux pour donner un groupement divalent $-OCH_2O-$, $-OCH_2CH_2O-$ ou $-CH=CH-CH=CH-$.

. $R_5$ et $R_6$ égaux ou différents représentent un hdyrogène, un groupement alcoyle ramifié ou non, saturé ou non, renfermant de 1 à 15 atomes de carbone, un groupement cycloalcoyle renfermant de 3 à 8 atomes de carbone et, en particulier, les radicaux H, Me, Et, i-Pr, n-heptyle, n-undécyle, n-pentadécyle, allyle, cyclohexyle. Enfin $R_5$ et $R_6$ peuvent fusionner pour donner un groupement polyméthylène $-(CH_2)_p-$ avec p pouvant prendre les valeurs de 2 à 7.

. $R_7$ représente un hydrogène, un groupement alcoyle ramifié ou non saturé ou non renfermant de 1 à 6 atomes de carbone, un groupement cycloalcoyle comportant de 3 à 8 atomes de carbone, un groupement aryle formé de 6 à 10 atomes de carbone substitué ou non, un groupement arylalcoyle renfermant de 7 à 15 atomes de carbone et, en particulier, les radicaux H, Me, Et, i-Pr, n-hexyle, allyle, cyclohexyle, phényle, benzyle, phénéthyle, méthoxy-4 phényle, diméthoxy-3,4 phénéthyle, chloro-4 phénéthyle.

. Les valeurs de m et n égales ou différentes peuvent varier de 1 à 4 inclus.

Ainsi que les sels organiques ou minéraux de I thérapeutiquement acceptables et, en particulier, un sel choisi parmi : chlorhydrate, bromhydrate, monophosphate, mésylate, hydrogénosulfate, succinate, maléate, fumarate, citrate, pamoate, salicylate, lactate, pyroglutamate, glutamate.

2) Composé selon la revendicaton 1 caractérisé en ce qu'il est choisi parmi les composés suivants :

- Chlorhydrate de dihydro-2,3 ((méthyl phénéthylamino)-3 propyl-3 4H-benzoxazine-1,3 one-4
- Chlorhydrate de dihydro-2,3 ((méthyl diméthoxy-3,4 phénéthylamino)-3 propyl)-3 4H-benzoxazine-1,3 one-4
- Dihydro-2,3 diméthoxy-6,7 ((méthyl diméthoxy-3,4 phénéthylamino)-3 propyl)-3 4H-benzoxazine-1,3

one-4 base
- Chlorhydrate de dihydro-2,3 diméthoxy-6,7 ((méthyl diméthoxy-3,4 phénéthylamino)-3 propyl)-3 4H-benzoxazine-1,3 one-4
- Bromhydrate de dihydro-2,3 diméthoxy-6,7 ((méthyl diméthoxy-3,4 phénéthylamino)-3 propyl)-3 4H-benzoxazine-1,3 one-4
- Hydrogénomaléate de dihydro-2,3 diméthoxy-6,7 ((méthyl diméthoxy-3,4 phénéthylamino)-3 propyl)-3 4H-benzoxazine-1,3 one-4
- Pamoate de dihydro-2,3 diméthoxy-6,7 ((méthyl diméthoxy-3,4 phénéthylamino)-3-propyl)-3 4H-benzoxazine-1,3 one-4
- Pyroglutamate de dihydro-2,3 diméthoxy-6,7 ((méthyl diméthoxy-3,4 phénéthylamino)-3 propyl)-3 4H-benzoxazine-1,3 one-4
- Chlorhydrate de dihydro-2,3 diméthoxy-6,7 ((méthyl diméthoxy-3,4 phénéthylamino)-4 butyl)-3 4H-benzoxazine-1,3 one-4
- Chlorhydrate de dihydro-2,3 diméthoxy-6,7 ((méthyl diméthoxy-3,4 phénéthylamino)-2 éthyl)-3 4H-benzoxazine-1,3 one-4
- Chlorhydrate de dihydro-2,3 diméthoxy-6,7 ((méthyl diméthoxy-3,4 benzylamino)-3 propyl)-3 4H-benzoxazine-1,3 one-4
- Chlorhydrate de dihydro-2,3 diméthoxy-6,7 ((méthoxy-4 phényl benzylamino)-3 propyl)-3 4H-benzoxazine-1,3 one-4
- Chlorhydrate de dihydro-2,3 diméthoxy-6,7 ((isopropyl diméthoxy-3,4 phénéthylamino)-3 propyl)-3 4H-benzoxazine-1,3 one-4
- Chlorhydrate de dihydro-2,3 diméthoxy-6,7 ((allyl diméthoxy-3,4 phénéthylamino)-3 propyl)-3 4H-benzoxazine-1,3 one-4
- Chlorhydrate de dihydro-2,3 diméthoxy-6,7 ((méthyl méthoxy-4 phénéthylamino)-3 propyl)-3 4H-benzoxazine-1,3 one-4
- Chlorhydrate de dihydro-2,3 diméthoxy-6,7 ((méthyl méthoxy-3 phénéthylamino)-3 propyl)-3 4H-benzoxazine-1,3 one-4
- Chlorhydrate de dihydro-2,3 diméthoxy-6,7 ((méthyl chloro-4 phénéthylamino)-3 propyl)-3 4H-benzoxazine-1,3 one-4
- Chlorhydrate de dihydro-2,3 diméthoxy-6,7 ((méthyl chloro-2 phénéthylamino)-3 propyl)-3 4H-benzoxazine-1,3 one-4
- Chlorhydrate de dihydro-2,3 diméthoxy-6,7 ((diméthyl-3,4 phénéthylamino)-3 propyl)-3 4H-benzoxazine-1,3 one-4
- Chlorhydrate de dihydro-2,3 diméthoxy-6,7 ((diméthyl-3,4 phénéthylamino)-3 éthyl)-3 4H-benzoxazine-1,3 one-4
- Chlorhydrate de dihydro-2,3 diméthoxy-6,7 ((benzyl diméthyl-3,4 phénéthylamino)-3 propyl)-3 4H-benzoxazine-1,3 one-4
- Chlorhydrate de dihydro-2,3 diméthoxy-6,7 ((méthyl phényl-3 propylamino)-3 propyl)-3 4H-benzoxazine-1,3 one-4
- Chlorhydrate de dihydro-2,3 diméthoxy-6,7 ((méthyl phényl-4 butylamino)-3 propyl)-3 4H-benzoxazine-1,3 one-4
- Chlorhydrate de dihydro-2,3 diméthoxy-6,7 ((méthyl phénéthylamino)-3 propyl)-3 4H-benzoxazine-1,3 one-4
- Chlorhydrate de dihydro-2,3 diméthoxy-6,7 méthyl-2 ((méthyl diméthoxy-3,4 phénéthylamino)-3 propyl)-3 4H-benzoxazine-1,3 one-4
- Chlorhydrate de dihydro-2,3 diméthoxy-6,7 n-heptyl-2 ((méthyl diméthoxy-3,4 phénéthylamino)-3 propyl)-3 4H-benzoxazine-1,3 one-4
- Chlorhydrate de dihydro-2,3 diméthoxy-6,7 n-undécyl-2 ((méthyl diméthoxy-3,4 phénéthylamino)-3 propyl)-3 4H-benzoxazine-1,3 one-4
- Chlorhydrate de dihydro-2,3 diméthoxy-6,7 diéthyl-2,2 ((méthyl diméthoxy-3,4 phénéthylamino)-3 propyl)-3 4H-benzoxazine-1,3 one-4
- Chlorhydrate de dihydro-2,3 diméthoxy-6,7 cyclohexyl-2 ((méthyl diméthoxy-3,4 phénéthylamino)-3 propyl)-3 4H-benzoxazine-1,3 one-4
- Chlorhydrate de dihydro-2,3 diméthoxy-6,7 ((méthyl diméthoxy-3,4 phénéthylamino)-3 propyl)-3 4H-benzoxazine-1,3 one-4 spiro-2 cyclohexane
- Chlorhydrate de dihydro-2,3 ((méthyl phénéthylamino)-3 propyl)-3 n-undécyl-2 4H-benzoxazine-1,3 one-4
- Chlorhydrate de dihydro-2,3 ((méthyl méthoxy-3 phénéthylamino)-3 propyl)-3 méthoxy-6-undécyl-2 4H-benzoxazine-1,3 one-4
- Chlorhydrate de dihydro-2,3 chloro-7 ((méthyl diméthoxy-3,4 phénéthylamino)-3 propyl)-3 4H-benzoxazine-1,3 one-4
- Chlorhydrate de dihydro-2,3 chloro-6 ((méthyl diméthoxy-3,4 phénéthylamino)-3 propyl)-3 4H-benzoxazine-1,3 one-4
- Chlorhydrate de dihydro-2,3 bromo-6 ((méthyl diméthoxy-3,4 phénéthylamino)-3 propyl)-3 4H-benzoxazine-1,3 one-4

- Chlorhydrate de dihydro-2,3 méthyl-6 ((méthyl diméthoxy-3,4 phénéthylamino)-3 propyl)-3 4H-benzoxazine-1,3 one-4
- Chlorhydrate de dihydro-2,3 méthyl-7 ((méthyl diméthoxy-3,4 phénéthylamino)-3 propyl)-3 4H-benzoxazine-1,3 one-4
- Chlorhydrate de dihydro-2,3 méthyl-8 ((méthyl diméthoxy-3,4 phénéthylamino)-3 propyl)-3 4H-benzoxazine-1,3 one-4
- Chlorhydrate de dihydro-2,3 méthoxy-6 ((méthyl diméthoxy-3,4 phénéthylamino)-3 propyl)-3 4H-benzoxazine-1,3 one-4
- Chlorhydrate de dihydro-2,3 méthoxy-7 ((méthyl diméthoxy-3,4 phénéthylamino)-3 propyl)-3 4H-benzoxazine-1,3 one-4
- Chlorhydrate de dihydro-2,3 méthoxy-8 ((méthyl diméthoxy-3,4 phénéthylamino)-3 propyl)-3 4H-benzoxazine-1,3 one-4
- Chlorhydrate de dihydro-2,3 méthoxy-8 méthyl-2 ((méthyl diméthoxy-3,4 phénéthylamino)-3 propyl)-3 4H-benzoxazine-1,3 one-4
- Chlorhydrate de dihydro-2,3 nitro-6 ((méthyl diméthoxy-3,4 phénéthylamino)-3 propyl)-3 4H-benzoxazine-1,3 one-4
- Chlorhydrate de dihydro-2,3 amino-6 ((méthyl diméthoxy-3,4 phénéthylamino)-3 propyl)-3 4H-benzoxazine-1,3 one-4
- Chlorhydrate de dihydro-2,3 acétamido-6 ((méthyl diméthoxy-3,4 phénéthylamino)-3 propyl)-3 4H-benzoxazine-1,3 one-4
- Chlorhydrate de dihydro-2,3 diméthylamino-6 ((méthyl diméthoxy-3,4 phénéthylamino)-3 propyl)-3 4H-benzoxazine-1,3 one-4
- Chlorhydrate de dihydro-2,3 diméthoxy-5,7 ((méthyl diméthoxy-3,4 phénéthylamino)-3 propyl)-3 4H-benzoxazine-1,3 one-4
- Chlorhydrate de dihydro-2,3 méthylènedioxy-6,7 ((méthyl diméthoxy-3,4 phénéthylamino)-3 propyl) 4H-benzoxazine-1,3 one-4
- Chlorhydrate de dihydro-2,3 ((méthyl diméthoxy-3,4 phénéthylamino)-3 propyl)-3 4H-Napht [2,3-e] oxazine-1,3 one-4
- Chlorhydrate de dihydro-2,3 diméthoxy-6,7 ((diméthoxy-3,4 benzylamino)-3 propyl)-3 4H-benzoxazine-1,3 one-4
- Chlorhydrate de dihydro-2,3 diméthoxy-6,7 ((méthyl (diméthoxy-3,4 phényl)-4 butylamino)-3 propyl)-3 4H-benzoxazine-1,3 one-4
- Chlorhydrate de dihydro-2,3 diméthoxy-6,7 ((diméthoxy-3,4 phénéthylamino)-4 butyl)-3 4H-benzoxazine-1,3 one-4
- Chlorhydrate de dihydro-2,3 diméthoxy-6,7 ((phényl-4 butyl amino)-3 propyl)-3 4H-benzoxazine-1,3 one-4
- Chlorhydrate de dihydro-2,3 [(phénéthylamino)-3 propyl]-3 4H-benzoxazine-1,3 one-4
- Chlorhydrate de dihydro-2,3 chloro-6 ((diméthoxy-3,4 phénéthylamino)-3 propyl)-3 4H-benzoxazine-1,3 one-4
- Chlorhydrate de dihydro-2,3 chloro-6 ((méthyl diméthoxy-3,4 phénéthylamino propyl)-3 méthyl-2 4H-benzoxazine-1,3 one-4
- Chlorhydrate de dihydro-2,3 chloro-6 ((diméthoxy-3,4 phénéthylamino)-3 propyl)-3 méthyl-2 4H-benzoxazine-1,3 one-4
- Hydrogénooxalate de dihydro-2,3 chloro-6 ((diméthoxy-3,4 phénéthylamino)-3 propyl)-3 n-heptyl-2 4H-benzoxazine-1,3 one-4
- Hydrogénooxalate de dihydro-2,3 chloro-6 ((méthyl diméthoxy-3,4 phénéthylamino)-3 propyl)-3 n-heptyl-2 4H-benzoxazine-1,3 one-4
- Chlorhydrate de dihydro-2,3 chloro-6,8 ((diméthoxy-3,4 phénéthylamino)-3 propyl)-3 4H-benzoxazine-1,3 one-4
- Chlorhydrate de dihydro-2,3 chloro-6,8 ((méthyl diméthoxy-3,4 phénéthylamino)-3 propyl)-3 4H-benzoxazine-1,3 one-4
- Chlorhydrate de dihydro-2,3 ((diméthoxy-3,4 phénéthylamino)-3 propyl)-3 nitro-6 4H-benzoxazine-1,3 one-4.

3) Procédé de préparation des composés chimiques selon les revendications 1 et 2 caractérisé en ce que l'on traite un salicylamide convenablement substitué de structure générale II par un composé carbonylé de formule III

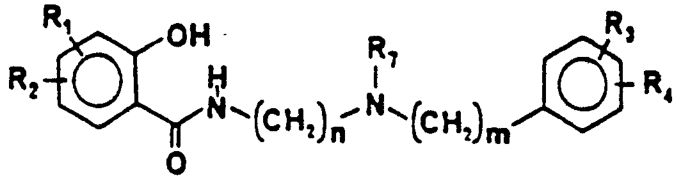

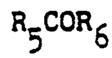

II

III

sous forme libre ou activée (cétal, trioxyméthylène, paraldéhyde, ou analogues) où $R_1$ à $R_7$ et n et m ont la même signification que dans la revendication 1 avec $R_5$, $R_6$ différent du radical aryle et $R_7$ différent de H.

4) Procédé de préparation selon la revendication 3 caractérisé en ce que la réaction de cyclisation est réalisée en milieu acide fort comme par exemple l'acide chlorhydrique gazeux, APTS, acide sulfurique dans un système de solvant : l'un aprotique comme le chloroforme ou l'acétate d'éthyle, l'autre protique comme un acide carboxylique et plus précisément l'acide acétique.

5) Procédé de préparation selon les revendications 3 et 4 caractérisé en ce que la réaction est réalisée à une température comprise entre 20 et 75° C.

6) Procédé de préparation des composés chimiques de formule générale I selon les revendications 1 et 2 caractérisé en ce que l'on condense une forme activée de la fonction alcool de la dihydro-2,3 hydroxy-n alkyl-3 4H-benzoxazine-1,3 one-4 substituée de formule IV :

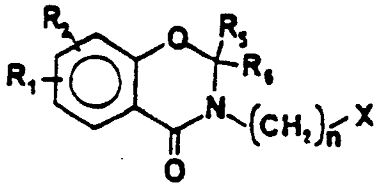

IV

avec une arylalkylamine de formule V :

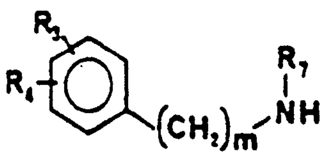

V

où $R_1$ à $R_7$, n et m ont la même signification que dans I et où X représente un halogène ou un radical aryle ou alkanesulfonate et plus précisément un chlore, brome, tosylate et méthanesulfonate.

7) Procédé de préparation des composés chimiques de formule générale I selon les revendications 1 et 2 caractérisé en ce que l'on condense en présence d'une base organique ou minérale le composé de formule générale I où $R_7$ = H avec un composé activé de formule $R_7$-Z où Z représente un halogène comme le chlore, le brome et l'iode ou de formule $R_7SO_4R_7$ où $R_7$ a la même signification que dans 1.

8) Procédé de préparation des composés chimiques de formule générale I selon les revendications 1 et 2 caractérisé en ce que l'on condense selon la réaction d'Eschweiler-Clarke un aldéhyde de formule $R_7CHO$ avec le composé de formule générale I où $R_7$=H en présence d'acide formique à une température comprise entre 25 et 100° C.

9) Procédé de préparation des composé chimiques de formule générale I selon les revendications 1 et 2 caractérisé en ce que l'on condense un chlorure d'acide salicylique convenablement substitué de formule générale VI :

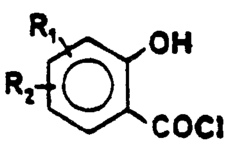

VI

avec une base de Shiff de formule générale VII :

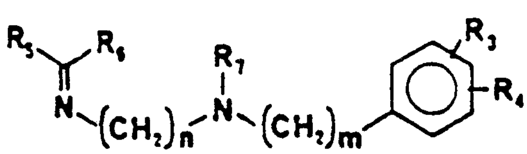

VII

où $R_1$ à $R_7$ ont la même signification que dans la revendication 1.

10) A titre de composé nouveau les intermédiaires selon la revendication 6 de formule générale IV :

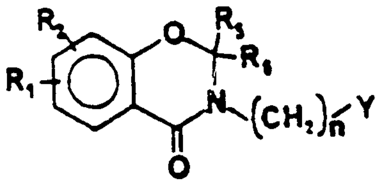

IV

où $R_1$, $R_2$, $R_5$, $R_6$, n ont la même signification que dans la revendication 1 et Y représente un halogène, un alkyl ou arylsulfonate.

11) Composé selon la revendication 10 caractérisé en ce que Y représente plus précisément un chlore, un brome ou le radical mésylate, tosylate.

12) A titre de médicaments, notamment utiles dans le traitement des troubles du système cardiovasculaire, et plus particulièrement comme bradycardisant, antiarythmique, antiangoreux et antiischémique les composés selon l'une des revendications 1 et 2.

13) Compositions pharmaceutiques caractérisées en ce qu'elles contiennent comme principe actif au moins un composé selon l'une des revendications 1 à 2 associé à un support pharmaceutique inerte.

Office européen des brevets

# RAPPORT DE RECHERCHE EUROPEENNE

Numero de la demande

EP 89 40 2425

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | FR-A-2 043 463 (SQUIBB)<br>* Revendications *<br>--- | 1,9,12,13 | C 07 D 265/22<br>C 07 D 265/12<br>C 07 D 498/04<br>A 61 K 31/535//<br>(C 07 D 498/04<br>C 07 D 317:00<br>C 07 D 265:00 ) |
| Y | FR-A-2 043 464 (SQUIBB)<br>* Page 6, formule VII; revendications *<br>--- | 10,11 | |
| Y | US-A-3 396 164 (U. TEOTINO)<br>* En entier *<br>--- | 1,10 | |
| A | CHEMICAL ABSTRACTS, vol. 104, no. 13, 31 mars 1986, page 733, résumé no. 109690s, Columbus, Ohio, US; & JP-A-60 169 470 (CHUGAI PHARMACEUTICAL CO. LTD) 02-09-1985<br>* Résumé *<br>----- | 1,9,12,13 | |
| | | | **DOMAINES TECHNIQUES RECHERCHES (Int. Cl.4)** |
| | | | C 07 D 265/00<br>C 07 D 498/00<br>A 61 K 31/00 |

Le présent rapport a été établi pour toutes les revendications

| Lieu de la recherche | Date d'achèvement de la recherche | Examinateur |
|---|---|---|
| LA HAYE | 12-12-1989 | CHOULY J. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant